# EUROPÄISCHE PATENTSCHRIFT

(11) **EP 1 107 809 B1**
(45) Veröffentlichungstag und Bekanntmachung des Hinweises auf die Patenterteilung: **03.09.2003**
(21) Anmeldenummer: 99952342.6
(22) Anmeldetag: 07.08.1999
(51) Int. Cl.: A61M 15/00

(54) **INHALATOR ZUM VERNEBELN VON FLÜSSIGKEITEN**
INHALATOR FOR ATOMIZING LIQUIDS
INHALATEUR POUR NEBULISER DES LIQUIDES

(30) Priorität: 26.08.1998 DE 19838711
(43) Veröffentlichungstag der Anmeldung: 20.06.2001
(73) Patentinhaber: Otto Schill GmbH & Co. KG, 70734 Fellbach (DE)
(72) Erfinder: DOETZ, Klaus-Joachim, D-07318 Saalfeld (DE); GESSLER, Tobias, D-35435 Wettenberg (DE); SCHILL, Immo, D-89150 Laichingen (DE); SCHMEHL, Thomas, D-35396 Giessen (DE)
(74) Vertreter: Patentanwalts-Partnerschaft, Rotermund + Pfusch + Bernhard
(86) Internationale Anmeldenummer: DE9902487
(87) Internationale Veröffentlichungsnummer: WO00012161

(56) Entgegenhaltungen:
- EP-A- 0 626 180
- WO-A-86/01731
- DD-A- 22 150
- US-A- 5 165 392
- US-A- 5 584 285

## Beschreibung

Die Erfindung betrifft einen Inhalator mit einer Einrichtung zum Vernebeln von Flüssigkeiten, die dann im Rahmen einer Heilbehandlung von einem Verwender des Inhalators eingeatmet werden. Derartige Inhalatoren sind z.B. aus der US 5 549 102, US 5 584 285, US 5 579 757 und US 5 655 520 bekannt.

Bei den Flüssigkeiten, die mit Hilfe eines solchen Inhalators vernebelt und als Aerosol-Nebel inhaliert werden sollen, handelt es sich üblicherweise um Medikamente, Heilmittel bzw. Wirkstoffe, die vorzugsweise als Lösungen, Suspensionen oder Emulsionen vorliegen. Diese Wirkflüssigkeit wird in ein Aufnahmegefäß des Inhalators eingebracht. Die Vernebelung erfolgt dann mit Hilfe eines Verneblers, der beispielsweise als Zerstäuberdüse oder als Ultraschallgenerator ausgebildet sein kann. Ein Ultraschallgenerator wandelt beispielsweise mit einer Frequenz von 1,7 MHz elektrische Energie in mechanische Schwingungen um, mit denen die zu vernebelnde Flüssigkeit beaufschlagt wird. Bei der Übertragung dieser Schwingungen auf die Inhalations- bzw. Heilmittellösungen lösen sich an der Oberfläche dieser Flüssigkeit ständig kleinste Tröpfchen ab, die einen lungengängigen Durchmesser aufweisen. Die durch Ultraschall aus der Flüssigkeit ausgelösten Tröpfchen bilden in einer Nebelkammer des Inhalators einen Aerosol-Nebel, der zum Inhalieren mit Frischluft vermischt wird und dabei ein gut alveolargängiges Inhalat bildet. Diese mit Aerosol-Nebel angereicherte Atemluft bzw. dieses Inhalat dringt in die feinsten Verästelungen der Lunge vor und erreicht dabei eine hohe Depositionsrate.

Beim Inhalieren atmet der Verwender über ein Mundstück eines an die Nebelkammer angeschlossenen Mündungselementes ein, wobei über eine Einatemöffnung Frischluft in die Nebelkammer eintritt und sich mit dem dort gebildeten Nebel vermischt und so die Atemluft mit dem Aerosol anreichert. Wenn der Verwender beim Ausatmen durch das Mundstück ausatmet, kommt es zu einer intensiven Durchmischung mit dem bereitgestellten Nebel und somit zu einem entsprechenden Austrag an ungenutzter Flüssigkeit. Ein derart hoher Verbrauch ist besonders bei teuren Medikamenten unerwünscht.

Alternativ kann der Verwender beim Ausatmen das Mundstück freigeben und dementsprechend frei in die Atmosphäre ausatmen. Auf diese Weise gelangt die Ausatemluft in die Umgebung des Verwenders. Da bei einem Atemzug nicht sämtliche Tröpfchen in den Atemwegen des Verwenders abgelagert werden, enthält auch die Ausatemluft noch eine nicht vernachlässigbare Menge an vernebelter Flüssigkeit. Bei einigen Anwendungsfällen solcher Inhalatoren bilden diese Restbestandteile der vernebelten Medikamente in der Ausatemluft kein Problem. Bei anderen Anwendungsfällen, bei denen Medikamente inhaliert werden, ist es wünschenswert oder sogar notwendig, daß die Wirkung und auch die Nebenwirkung des Medikaments auf den Verwender beschränkt bleibt. Es sollte kein Wirkstoffaustrag in die Umgebung erfolgen, da dieser je nach verwendetem Medikament eine Gefahr für die Umwelt des Verwenders darstellen könnte. Insbesondere Schwangere, Immungeschwächte, Allergiker und Kinder sind durch die Medikamentenreste, die frei in die Umwelt gelangen, in ihrer Gesundheit gefährdet.

Aus der EP 0 626 180 A1 sowie aus der US 5 584 285 ist jeweils ein Inhalator gemäß dem Oberbegriff des Anspruchs 1 bekannt. Ein derartiger Inhalator besitzt einen Handapparat mit einem Griffkörper, einer Nebelkammer und einem Mündungselement. Der zylindrische Griffkörper enthält in einem oberen Abschnitt ein nach oben offenes Aufnahmegefäß zur Aufnahme einer zu vernebelnden Flüssigkeit. In den Griffkörper integriert ist dabei eine Düse zur Zerstäubung der Flüssigkeit. Die Nebelkammer weist eine zylindrische Form auf und ist. mit ihrem unteren Ende an den Griffkörper angeschlossen und kommuniziert so mit dem Aufnahmegefäß. An ihrem oberen Ende enthält die Nebelkammer eine Einatemöffnung, durch die beim Ansaugen Frischluft aus der Umgebung in die Nebelkammer eintritt. Zwischen ihren Enden ist seitlich an die Nebelkammer das Mündungselement angeschlossen, das eine Mündungsöffnung sowie eine Ausatemöffnung enthält, durch die beim Ausatmen Ausatemluft in die Umgebung austritt. In der Einatemöffnung ist ein Einatemventil angeordnet, das beim Einatmen öffnet und beim Ausatmen schließt. In der Ausatemöffnung ist ein Ausatemventil angeordnet, das beim Einatmen schließt und beim Ausamen öffnet. Das Mündungselement weist in einem mittleren Bereich einen sich zur Mündungsöffnung hin verjüngenden, trichterförmigen Abschnitt auf, in dem die Ausatemöffnung angeordnet ist. Der Handapparatinnenraum ist zumindest bis zur Ausatemöffnung gas- und druckdicht ausgebildet, derart, daß bei geschlossenem Einatemventil die in Richtung auf den Handapparatinnenraum strömende Ausatemluft zur Ausatemöffnung hin ausweichen muß, wobei die Ausatemluft im wesentlichen nicht in den Handapparatinnenraum stromab der Ausatemöffnung eindringt.

Aus der US 5 165 392 ist ein weiterer Inhalator bekannt, der ein zylindrisches Gehäuse besitzt, das an einem Ende einen ersten Anschluß zur Anbringung eines Mundstücks besitzt und an seinem anderen Ende einen zweiten Anschluß für ein Filterelement aufweist. In seiner Längsrichtung ist das Gehäuse in zwei voneinander getrennte Kammern unterteilt, nämlich in eine obere Durchströmungskammer und eine untere Durchströmungskammer. Im Gehäuse ist ein Vernebler untergebracht, der beide Kammern durchdringt, wobei eine Austrittsöffnung des Verneblers mit der oberen Durchströmungskammer kommuniziert. An der dem Filter zugewandten Seite besitzt die obere Durchströmungskammer ein Einströmventil, während die untere Durchströmungskammer an dieser Stelle ein Ausströmventil aufweist. An der dem Mundstück zugewandten Seite sind die beiden Kammern offen und können miteinander kommunizieren.

Die vorliegende Erfindung beschäftigt sich mit dem Problem, einen gattungsgemäßen Inhalator dahingehend auszugestalten, daß er mit geringen Verlusten an der zu vernebelnden Flüssigkeit arbeitet und eine besonders effektive Inhalation ermöglicht.

Darüber hinaus beschäftigt sich die vorliegende Erfindung auch mit dem Problem, einen Inhalator so auszugestalten, daß eine Kontamination der Umwelt während der Anwendung vermieden wird und der Inhalator somit zum Inhalieren auch potentiell toxischer Medikamente geeignet ist.

Zur Lösung der vorstehend genannten Probleme wird ein Inhalator mit den Merkmalen des Anspruches 1 vorgeschlagen.

Die Erfindung beruht zum einen auf dem allgemeinen Gedanken, daß der Verwender auch durch den Inhalator ausatmet, wobei der Inhalator eine Ausatemöffnung aufweist, die insbesondere für den Fall, daß mit toxischen Medikamenten inhaliert werden soll, mit einem geeigneten Filter (Ausatemfilter) versehen ist. Auf diese Weise wird die Ausatemluft vor ihrem Austritt in die Umgebung des Verwenders gefiltert. Somit können Rückstände des Medikamentes in der Ausatemluft nicht mehr in die Umgebung des Verwenders gelangen, sondern verbleiben im Inhalator bzw. im Filter. Die Gefahr eines unerwünschten Kontaktes mit dem Medikament wird daher vermieden.

Die Erfindung beruht zum anderen auch auf dem allgemeinen Gedanken, im Handapparat die Strömung der Ausatemluft so zu führen, daß möglichst wenig des für den nächsten Einatemvorgang bereitgestellten Inhalats von der Ausatemströmung mitgerissen und durch die Ausatemöffnung in die Umgebung austritt oder gegebenenfalls im Filter ausgefiltert wird. Denn der ausgefilterte Wirkstoff wäre in der Regel verloren. Diese Strömungsführung wird zum einen durch die Strömungsleitmittel eingeleitet und zum anderen durch die Anordnung und Orientierung der Ausatemöffnung unterstützt. Auf diese Weise ergeben sich beim Einatmen und beim Ausatmen unterschiedliche Durchströmungswege im Inhalator, wobei durch eine entsprechende Ausgestaltung der Nebelkammer und des Mündungselementes sich diese Strömungswege so ausgestalten lassen, daß durch die Einatemströmung eine relativ große Verwirbelung mit dem in der Nebelkammer erzeugten Flüssigkeits-Nebel erzielt wird, während die Ausatemströmung eine erheblich geringere Verwirbelung mit dem Nebel erzeugt. Durch diese Maßnahme wird gewährleistet, daß beim Ausatmen möglichst geringe Mengen des noch nicht inhalierten Medikamenten-Nebels in die Umgebung oder in das Filter gelangen. Dies ist insbesondere bei teuren Medikamenten von besonderem Interesse.

Beim erfindungsgemäßen Inhalator ist in der Einatemöffnung ein Einatemventil angeordnet, das beim Einatmen öffnet und beim Ausatmen schließt, und in der separat ausgebildeten Ausatemöffnung ist stromauf oder stromab des Filters ein Ausatemventil angeordnet, das beim Einatmen schließt und beim Ausatmen öffnet. Aufgrund der erfindungsgemäßen Anordnung und Orientierung der Ausatemöffnung kann die Ausatemströmung im Mündungsbereich quer zur Einatemströmung ausweichen bzw. durch die dort angeordnete Ausatemöffnung aus dem Handapparat entweichen. Hierbei wird das sogenannte "Flaschenhals-Prinzip" ausgenutzt, wonach eine frontal auf eine Flaschenöffnung gerichtete (Luft-)Strömung nur geringfügig in die Flasche eindringen kann, im wesentlichen jedoch quer zur Flaschenöffnung abgelenkt wird. Beim erfindungsgemäßen Inhalator hat die Ausatemströmung bis zur Austrittsöffnung die entgegengesetzte Richtung wie die Einatemströmung. Da das sich hinsichtlich der Einatemströmung stromauf an die Ausatemöffnung angrenzende Innere des Handapparates aufgrund des beim Ausatmen geschlossenen Einatemventils druckdicht ist, kann die Ausatemströmung im wesentlichen nicht in dieses druckdicht abgeschlossene Innere des Handapparates eindringen, sondern wird quer dazu durch die Ausatemöffnung abgelenkt. Dies hat zur Folge, daß kaum eine Durchmischung von Ausatemluft mit frischem Inhalat stattfindet. Demnach bewirkt die erfindungsgemäße Anordnung und Orientierung der Ausatemöffnung in Verbindung mit den Ventilen lediglich einen minimalen Mitnahmeeffekt hinsichtlich des für einen nächsten Einatemvorgang vorbereiteten Inhalats.

Damit auch für den Schließvorgang des Einatemventils ein Medikamentenaustrag in die Umwelt effektiv verhindert werden kann, ist entsprechend einer Weiterbildung des Inhalators ein zusätzliches Filter (Einatemfilter) am Einatemventil bezüglich einer Einatemströmung stromauf angebracht.

Um die Ausgestaltung unterschiedlicher Strömungswege für das Einatmen und für das Ausatmen zu unterstützen, ist beim erfindungsgemäßen Inhalator die Ausatemöffnung vorzugsweise zwischen der Einatemöffnung und der Mündungsöffnung des Mündungselementes angeordnet.

Bei einer besonders vorteilhaften Ausführungsform des erfindungsgemäßen Inhalators kann die Ausatemöffnung relativ nahe der Mündungsöffnung des Mündungselementes angeordnet sein, wobei dann der Strömungsweg für den Ausatemvorgang erheblich kürzer ist und somit deutlich weniger Einfluß auf den dann relativ weit entfernten Nebel in der Nebelkammer hat als der längere, durch die Nebelkammer geführte Strömungsweg für den Einatemvorgang. Darüber hinaus wird bei dieser Ausführungsform das Volumen zwischen der Mündungsöffnung des Mündungselements erheblich minimiert. Dadurch befindet sich in diesem Totraum zu Beginn der Ausatmung auch weniger Inhalat, das in der nachfolgenden Ausatmung über den Ausatemfilter verloren geht.

Gemäß einer besonders effektiv arbeitenden Ausführungsform ist das der Einatemströmung ausgesetzte Innere des Handapparates so ausgestaltet, daß sich bis zur Mündungsöffnung eine laminare Einatemströmung ausbildet. Dies wird insbesondere durch einen entlang des Strömungsweges abnehmenden Druck mittels eines abnehmenden Strömungsquerschnittes erreicht. Aufgrund der laminaren Strömung kommt es an den Innenwänden des Handapparates nur zu geringen Ablagerungen von Tröpfchen, so daß eine große Menge an Nebel-Tröpfchen inhaliert werden kann.

Um die Durchmischung der Ausatemluft mit dem Nebel in der Nebelkammer weiter zu reduzieren, kann zwischen der Einatemöffnung und der Ausatemöffnung zusätzlich ein Zwischenventil angeordnet sein, das parallel zum Einatemventil beim Einatmen öffnet und beim Ausatmen schließt. Bei einer entsprechend Anordnung dieses Zwischenventils kann die Nebelkammer bzw. der den Nebel enthaltende Bereich der Nebelkammer beim Ausatmen nicht mehr angeströmt bzw. durchströmt werden. Eine Durchmischung der Ausatemluft mit dem Nebel in der Nebelkammer wird dadurch weitestgehend verhindert, so daß im wesentlichen ausschließlich die in der Ausatemluft enthaltenen Nebel-Tröpfchen im Filter ausgefiltert werden. Zwar wird bei dieser Ausführungsform ein minimaler Ausstoß an neuem, für weitere Einatemvorgänge vorgesehener Aerosol-Nebel erreicht, jedoch bildet das Zwischenventil ein Strömungshindernis, an dem sich die vernebelte Flüssigkeit beim Einatmen ablagert und so die Effektivität des Inhalators insoweit reduziert.

Bei einer besonders vorteilhaften Ausführungsform des erfindungsgemäßen Inhalators ist der Strömungsweg des Inhalators vom Ort der Erzeugung im Aufnahmegefäß bis zur Mündungsöffnung des Mündungselements jedoch ohne Strömungshindernisse, wie z.B. das vorgenannte Zwischenventil, ausgestaltet. Durch das Fehlen von Strömungshindernissen, wie eines solchen Zwischenventils, kann die Ausbildung der laminaren Einatemströmungen besser gewährleistet werden. Darüber hinaus wird eine Abscheidung der vernebelten Flüssigkeit beispielsweise an einem derartigen Zwischenventil als Strömungshindernis vermieden und somit die Effektivität des Inhalators erhöht. Weiterhin macht sich die Hindernislosigkeit für den Verwender dahingehend vorteilhaft bemerkbar, daß dieser geringere Atemwiderstände zu überwinden hat und folglich eine geringere Atemarbeit der Inhalation leisten muß. Durch das oben erwähnte "Flaschenhals-Prinzip" ist dabei gewährleistet, daß auch ohne ein Zwischenventil praktisch keine Durchmischung von Ausatemluft mit frischem Inhalat stattfinden kann.

Weitere wichtige Merkmale und Vorteile des erfindungsgemäßen Inhalators ergeben sich aus den Unteransprüchen, aus den Zeichnungen und aus der zugehörigen Figurenbeschreibung anhand der Zeichnung.

Es versteht sich, daß die vorstehend genannten und die nachstehend noch zu erläuternden Merkmale nicht nur in der jeweils angegebenen Kombination, sondern auch in anderen Kombination oder in Alleinstellung verwendbar sind, ohne den Rahmen der vorliegenden Erfindung zu verlassen.

Ein bevorzugtes Ausführungsbeispiel der Erfindung ist in den Zeichnungen dargestellt und wird in der nachfolgenden Beschreibung näher erläutert. Es zeigen, jeweils schematisch,
- Fig. 1: eine teilweise geschnittene Seitenansicht auf einen Handapparat eines erfindungsgemäßen Inhalators beim Einatmen und
- Fig. 2: eine Ansicht wie in Fig. 1, jedoch beim Ausatmen.

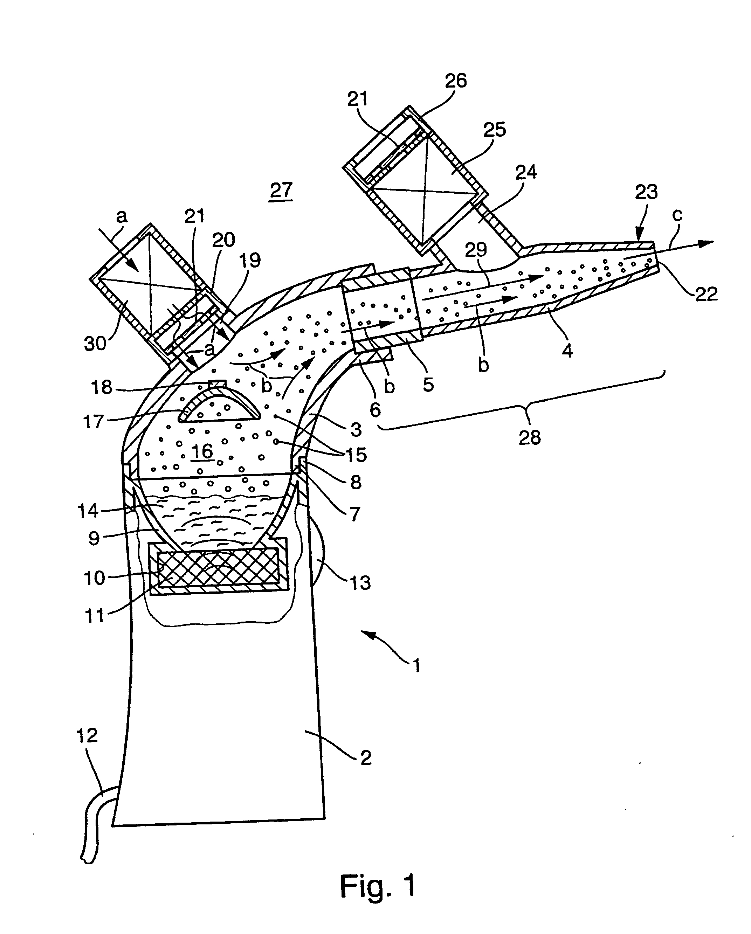

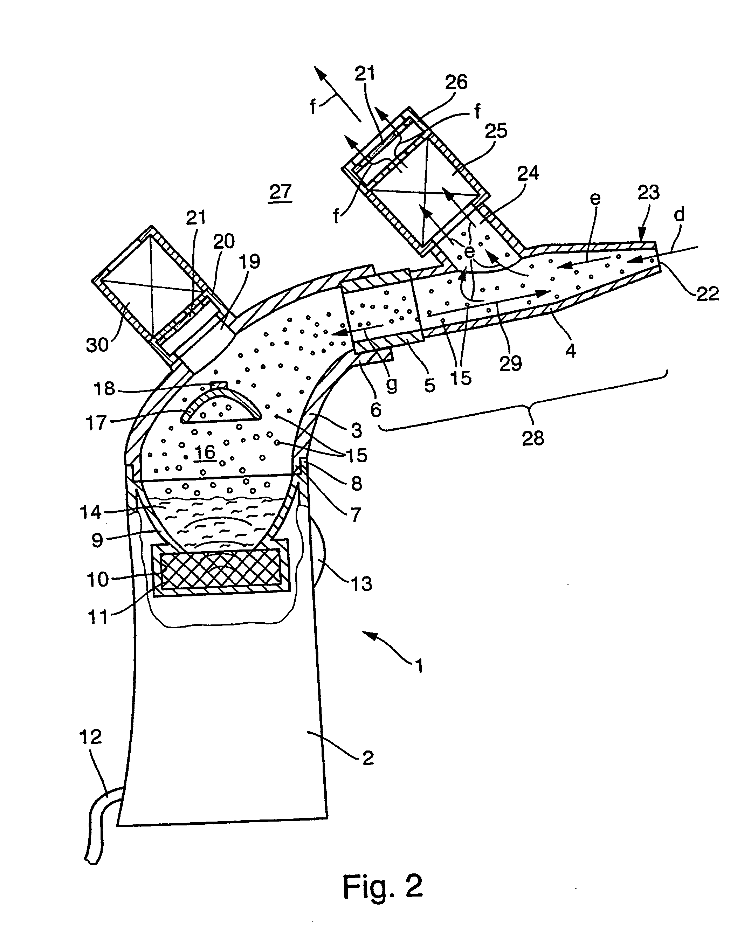

Entsprechend den Fig. 1 und 2 weist ein erfindungsgemäßer Inhalator einen Handapparat 1 auf, der im dargestellten Ausführungsbeispiel im wesentlichen aus drei Bauelementen bzw. Bauteilgruppen aufgebaut ist, nämlich aus einem Griffkörper 2, aus einer Nebelkammer 3 und aus einem Mündungselement 4.

In der dargestellten Ausführungsform sind die einzelnen Bauelemente mit Hilfe von Steckverbindungen aneinander angeschlossen, die jeweils durch Stutzen 5 bzw. 7 und Aufnahmen 6 bzw. 8 gebildet sind. Die Stutzen 5 und 7 sowie die Aufnahmen 6 und 8 sind dabei vorzugsweise mit zylindrischem, insbesondere kreiszylindrischem, Querschnitt ausgebildet, wodurch die Anschlüsse einfach herstellbar und auf einfache Weise abdichtbar sind. Beispielsweise können die Stutzen-Aufnahme-Verbindungen nach Art eines Bajonett-Verschlusses ausgebildet sein. Zur Abdichtung der Verbindungen zwischen Stutzen 5 bzw. 7 und Aufnahme 6 bzw. 8 sind die radial aneinander anliegenden Kontaktflächen der Stutzen 5 bzw. 7 und der Aufnahmen 6 bzw. 8 als radial wirkende Dichtflächen ausgebildet. Ebenso können bei einer anderen Ausführungsform andere Dichtelemente wie z.B. O-Ringe zum Abdichten der Verbindungen verwendet werden.

Der Griffkörper 2 ist in seinem oberen Abschnitt aufgebrochen dargestellt. Im Inneren des Griffkörpers 2 ist ein Aufnahmegefäß 9 angeordnet, das nach oben offen ausgebildet ist. In einer unteren Wandung bzw. im Boden 10 enthält das Aufnahmegefäß 9 einen als Ultraschallgenerator 11 ausgebildeten Vernebler. Zur Versorgung des Ultraschallgenerators 11 mit elektrischer Energie ist der Griffkörper 2 über ein Kabel 12 mit einem nicht dargestellten Basisgerät verbunden. Des weiteren weist der Griffkörper 2 einen Schalter 13 auf, bei dessen Betätigung der Ultraschallgenerator 11 mit elektrischer Energie versorgt und aktiviert wird. Der Griffkörper 2 ist ergonomisch gestaltet, derart, daß er mit einer Hand vom Verwender bequem gehalten werden kann, wobei dann der Schalter 13 mit einem Finger, vorzugsweise mit dem Daumen, der den Griffkörper 2 haltenden Hand leicht zugänglich und betätigbar ist. Alternativ kann auch ein Ein/Aus-Schalter am Basisgerät vorgesehen sein.

Zur Vorbereitung einer Inhalation wird das Aufnahmegefäß 9 mit einer zu vernebelnden Flüssigkeit 14 befüllt, die ein Medikament, Wirkstoff oder (Natur-)Heilmittel oder eine Medikament-, Wirkstoff- oder (Natur-)Heilmittel-Lösung sein kann. Zum Auffüllen des Aufnahmegefäßes 9 wird die Nebelkammer 3 zweckmäßigerweise vom Griffkörper 2 abgenommen.

Die Vernebelungseinrichtung 11 des erfindungsgemäßen Inhalators arbeitet nach dem Prinzip der Direktvernebelung, bei dem die zu vernebelnde Flüssigkeit 14 direkt mit dem Ultraschallgenerator 11 in Kontakt kommt. Andere Inhalatoren weisen Vernebelungseinrichtungen auf, die nach dem Prinzip der Indirektvernebelung arbeiten, bei dem die zu vernebelnde Flüssigkeit indirekt über eine Wandung oder über eine Übertragungsflüssigkeit mit dem Ultraschallgenerator 11 in Kontakt ist.

Durch die Betätigung des Schalters 13 wird der Ultraschallgenerator 11 aktiviert, so daß dieser auf die zu vernebelnde Flüssigkeit 14 einwirkt, wobei er die ihm zugeführte elektrische Energie in Form mechanischer Schwingungen im Ultraschallfrequenzbereich abgibt und direkt in die zu vernebelnde Flüssigkeit 14 einleitet. Diese Ultraschallschwingungen bewirken in der Flüssigkeit 14, -daß sich an deren Oberfläche kleine Tröpfchen 15 ablösen, die oberhalb der Flüssigkeit 14 und somit oberhalb des Aufnahmegefäßes 9 einen Flüssigkeitsnebel 16 erzeugen. Die auf diese Weise erzeugten Tröpfchen 15 weisen dabei regelmäßig einen Außendurchmesser von durchschnittlich 4 um auf und sind daher zum Inhalieren gut geeignet.

In der Nebelkammer 3 ist eine Prallplatte 17 angeordnet, die beispielsweise über einen Steg 18 mit der Nebelkammer 3 verbunden ist. Die Prallplatte 17 bewirkt, daß sich Tröpfchen 15 mit größerer kinetischer Energie, insbesondere Tröpfchen 15 mit ungünstig großem Außendurchmesser bei ihrem Aufstieg nach oben an der Prallplatte 17 niederschlagen und wieder nach unten in das Aufnahmegefäß 9 abtropfen. Auf diese Weise werden diese für einige Inhalationsanwendungen ungünstigen Tröpfchen 15 mit relativ großem Außendurchmesser durch die Prallplatte 17 aus dem Nebel 16 ausgefiltert.

Bei anderen Ausführungsformen kann zur Erzeugung eines Nebels 16 mit größeren Tröpfchen 15 die Prallplatte 17 anders ausgestaltet sein oder sogar entfallen.

In der Nebelkammer 3 ist im wesentlichen oberhalb des Aufnahmegefäßes 9 eine Einatemöffnung 19 angeordnet, die mit einem Einatemventil 20 versehen ist. Das Einatemventil 20 enthält eine bewegliche Ventilplatte 21, die sich je nach den am Einatemventil 20 herrschenden Druckverhältnissen im Einatemventil 20 verstellt und dadurch den Lufteintritt in die Nebelkammer 3 durch die Einatemöffnung 19 dementsprechend öffnet oder schließt. In die Einatemöffnung 19 ist vor dem zugehörigen Einatemventil 20 ein Einatemfilter 30 angebracht, das einen Austritt von Inhalat durch die Einatemöffnung 19 während des Schließens des Einatemventils 20 in die Umgebung 27 verhindert. Während die Nebelkammer 3 an ihrem einen, unteren Ende über die Steckverbindung 7, 8 an den Griffkörper 2 angeschlossen ist, befindet sich an ihrem anderen, oberen Ende das Mündungselement 4, das im dargestellten Ausführungsbeispiel über die Steckverbindung 5, 6 an die Nebelkammer 3 angeschlossen ist. Die Nebelkammer 3 weist eine gekrümmte Form auf, so daß eine Normale einer Ebene durch die Öffnung am unteren Ende, d.h. im Bereich des Anschlusses an den Griffkörper 2, etwa um 100° ± 10° gegenüber der einer Normalen einer Ebene durch die Öffnung am oberen Ende, d.h. im Bereich des Anschlusses an das Mündungselement 4, geneigt ist.

Das Mündungselement 4 ist in Form eines T-Stückes ausgebildet und weist an einem dem Stutzen 5 gegenüberliegenden Ende eine Mündungsöffnung 22 auf. Die Mündungsöffnung 22 bzw. der diese enthaltende Abschnitt des Mündungselementes 4 ist in Form eines Mundstückes 23 ausgebildet, durch das Verwender des Inhalators ein- und ausatmet. Im Inneren des Handapparates 1 ist unmittelbar an die Mündungsöffnung 22 anschließend ein Mündungsbereich 28 ausgebildet, der mit einer geschweiften Klammer hervorgehoben ist. Im Mündungsbereich 28 beim Einatmen wird eine auf die Mündungsöffnung 22 gerichtete Strömung ausgerichtet, derart, daß im Mündungsbereich 28 bereits die Strömungsrichtung vorliegt, mit der die Strömung durch die Mündungsöffnung 22 hindurchströmt. Diese als "Durchströmungsrichtung" bezeichnete Richtung ist durch einen Pfeil 29 symbolisiert.

Zwischen dem Stutzen 5 und der Mündungsöffnung 22 weist das Mündungselement 4 eine Ausatemöffnung 24 auf, in der ein Ausatemventil 26 und insbesondere bei der Verneblung toxischer Flüssigkeiten ein Filter 25 angeordnet sind. Das Filter 25 ist für die Tröpfchen 15 der vernebelten Flüssigkeit 14 undurchlässig. Das Ausatemventil 26 ist in entsprechender Weise ausgebildet wie das Einatemventil 20 und weist daher ebenfalls eine bewegliche Platte 21 auf, die sich je nach den am Ausatemventil 26 herrschenden Druckverhältnissen im Ausatemventil 26 verstellt und dadurch einen Luftaustritt aus dem Mündungselement 4 durch die Ausatemöffnung 24 und durch das Filter 25 dementsprechend ermöglicht oder sperrt.

Im Unterschied zu der dargestellten Ausführungsform kann die Reihenfolge, wie das Filter 25 und das Ausatemventil 26 in Strömungsrichtung aufeinander folgen, auch umgekehrt sein.

Anstelle der beispielhaft dargestellten Ventile 20, 26 können auch andere, insbesondere nach dem Rückschlag-Prinzip arbeitende Ventile verwendet werden.

Das Mündungselement 4 ist vorzugsweise abnehmbar an der Nebelkammer 3 angebracht. Ebenso ist die Nebelkammer 3 bevorzugt abnehmbar am Griffkörper 2 angebracht. Die Mündungsöffnung 22 ist hier als Mundstück 23 ausgebildet. Ebenso ist eine Ausführungsform möglich, bei der die Mündungsöffnung 22 als Mund/Nasenmaske ausgebildet ist. Außerdem können das Mundstück 23 oder die Mund/Nasemaske vorzugsweise abnehmbar am Mündungselement 4 angebracht sein.

Der Handapparat 1 des erfindungsgemäßen Inhalators arbeitet wie folgt:

Beim Einatmen saugt der Verwender entsprechend Fig. 1 mit seinem Mund über das Mundstück 23 Frischluft aus einer Umgebung 27 an. Während dieses Einatemvorganges wird im gesamten durchströmten Raum bzw. im kommunizierend verbundenen Inneren des Handapparates 1 ein Unterdruck gegenüber der Umgebung 27 ausgebildet, so daß sich die Ventilplatten 21 des Einatemventils 20 und des Ausatemventils 26 - wie in Fig. 1 dargestellt - in Richtung auf den Unterdruck verstellt haben. Dabei ist das Einatemventil 20 geöffnet und das Ausatemventil 26 geschlossen. Die Frischluft tritt dabei entsprechend den Pfeilen a durch das Einatemfilter 30, durch das Einatemventil 20 und durch die Einatemöffnung 19 in die Nebelkammer 3 ein. Aufgrund der dabei in der Nebelkammer 3 entstehenden Verwirbelung vermischt sich die Frischluft mit dem in der Nebelkammer 3 ausgebildeten Nebel 16. Die sich dabei ausbildende, mit Aerosol angereicherte Einatemluft strömt dann entsprechend den Pfeilen b von der Nebelkammer 3 in das Mündungselement 4. Vom Mündungselement 4 tritt die Einatemluft bzw. das Inhalat dann entsprechend dem Pfeil c aus der Mündungsöffnung 22 aus und wird direkt den Atemwegen des Verwenders zugeleitet. Dabei ist deutlich zu sehen, daß die Einatemluft im gesamten Mündungsbereich 28 in der Durchströmungsrichtung 29 strömt. Im Mündungsbereich 28 verlaufen daher die Strömungspfeile b und c parallel zur Durchströmungsrichtung 29.

Aufgrund des über dem Strömungsweg abnehmenden Durchströmungsquerschnittes fällt auch ständig der Druck weiter ab. Wobei dies und die Formgebung des durchströmten Inneren die Ausbildung einer laminaren Einatemströmung begünstigt.

Beim Ausatmen entsprechend Fig. 2 bläst der Verwender über das Mundstück 23 Atemluft entsprechend dem Pfeil d in das Mündungselement 4 ein. Dabei bildet sich in den mit der Mündungsöffnung 22 kommunizierenden Strömungsräumen des Handapparates 1 ein höherer Druck als in der Umgebung 27 aus, so daß sich die Ventilplatten 21 dementsprechend in Richtung auf den niedrigeren Druck verstellen. Entsprechend den Ventilstellungen in Fig. 2 ist das Einatemventil 20 daher geschlossen und das Ausatemventil 26 geöffnet.

Die Ausatemluft des Verwenders enthält noch immer Tröpfchen 15, die von den Atemwegen des Verwenders nicht aufgenommen worden sind.

Beim Ausatmen folgt die Strömung aufgrund der Ausatemventilstellung im wesentlichen den Pfeilen e und durchdringt das Filter 25, wobei die Rückstände der vernebelten Flüssigkeit 14 in der Ausatemluft im Filter 25 ausgefiltert werden. Nach dem Filter 25 tritt die von der Flüssigkeit 14 gereinigte Ausatemluft durch das Ausatemventil 26 entsprechend den Pfeilen f in die Umgebung 27 aus.

Hier wird die Funktionsweise des weiter oben geschilderten "Flaschenhals-Prinzips" deutlich. Im ersten Teil des Verlaufes der Ausatemströmung strömt die Ausatemluft genau entgegen der vorher beschriebenen Einatemströmung. Die Pfeile d und e verlaufen daher stromauf etwa bis zur Ausatemöffnung 24 parallel zur Durchströmungsrichtung 29. Da das Einatemventil 20 während des Ausatmens geschlossen und somit der Handapparatinnenraum stromab der Ausatemöffnung 24 luft- und druckdicht ist, gelangt aufgrund des "Flaschenhals-Prinzips" nur ein geringer Anteil der Ausatemluft in die Nebelkammer 3, was durch den Pfeil g angedeutet ist. Im übrigen muß die Ausatemströmung ausweichen, was durch die geneigt zur Durchströmungsrichtung 29 angeordnete Ausatemöffnung 24 möglich ist. Durch diese Ablenkung der Ausatemluft verläuft die Ausatemströmung in einem zweiten Teil beispielsweise mit einem Winkel von etwa 60° geneigt zur Durchströmungsrichtung 29. Dementsprechend sind die Strömungspfeile e und f in diesem Teil im wesentlichen um etwas 60° geneigt bezüglich der Durchströmungsrichtung 29.

Um das Flaschenhals-Prinzip effektiv ausnutzen zu können, ist der durchströmbare Innenraum des Handapparates 1 gasund druckdicht, z.B. bis 500 mbar, ausgebildet. Durch die gewählte Anordnung der Ventile 20 und 26 und der Filter 25 und 30 wird gewährleistet, daß beim Ausatmen keine Tröpfchen 15 der Flüssigkeit 14 in die Umgebung 27 gelangen können, so daß der erfindungsgemäße Inhalator auch für die Verwendung potentiell toxischer Medikamente geeignet ist. Durch die vorgenannte Dichtigkeit des Handapparates 1 werden auch gefährliche Leckströmungen vermieden.

Durch die gewählte Orientierung und durch die Anordnung der Ausatemöffnung 24 relativ nahe an der Mündungsöffnung 22 wird gewährleistet, daß keine oder nur eine geringfügige Durchmischung der Ausatemluft mit dem in der Nebelkammer 3 ausgebildeten Nebel 16 erfolgt. Auf diese Weise kann vermieden werden, daß unnötig viel Tröpfchen 15 des unter Umständen teuren Medikamentes (14) in das Filter 25 gelangen.

Bei einer anderen Ausführungsform kann das Mundstück 23 abnehmbar am Mündungselement 4 angebracht sein, wobei dann insbesondere ein Austausch gegen eine Mund/Nasen-Maske möglich ist, beispielsweise um ein Inhalieren durch die Nase zu ermöglichen.

Bei einer anderen Ausführungsform kann zwischen der Nebelkammer 3 und dem Mündungselement 4, vorzugsweise nahe an der Ausatemöffnung 24 ein zusätzliches, hier nicht dargestelltes Zwischenventil angeordnet sein, das parallel zum Einatemventil 20 arbeitet, das heißt das Zwischenventil öffnet beim Einatmen und schließt beim Ausatmen. Dieses Zwischenventil verhindert effektiv eine Durchmischung der Ausatemluft mit dem Nebel 16 der Nebelkammer 3, um auf diese Weise die Menge der im Filter 25 abgelagerten Flüssigkeits-Tröpfchen 15 auf die in der Atemluft enthaltenden Tröpfchen 15 zu beschränken.

## Patentansprüche

1. Inhalator mit einer Einrichtung zum Vernebeln von Flüssigkeiten, mit folgenden Merkmalen:
A. ein Handapparat (1) des Inhalators weist einen Griffkörper (2), eine Nebelkammer (3) und ein Mündungselement (4) auf,
B. der Griffkörper (2) enthält in einem oberen Abschnitt ein nach oben offenes Aufnahmegefäß (9) zur Aufnahme einer zu vernebelnden Flüssigkeit (14),
C. der Griffkörper (2) enthält zudem einen Vernebler (11) zum Vernebeln der Flüssigkeit (14),
D. die Nebelkammer (3) ist mit ihrem unteren Ende an den Griffkörper (2) angeschlossen und kommuniziert mit dem Aufnahmegefäß (9),
E. die Nebelkammer (3) enthält eine Einatemöffnung (19), durch die beim Ansaugen Frischluft aus der Umgebung (27) in die Nebelkammer (3) eintritt,
F. das Mündungselement (4) enthält eine Mündungsöffnung (22) sowie eine Ausatemöffnung (24), durch die beim Ausatmen Ausatemluft in die Umgebung austritt,
G. in der Einatemöffnung (19) ist ein Einatemventil (20) angeordnet, das beim Einatmen öffnet und beim Ausatmen schließt,
H. in der Ausatemöffnung (24) ist ein Ausatemventil (26) angeordnet, das beim Einatmen schließt und beim Ausatmen öffnet,
I. der Handapparatinnenraum ist zumindest bis zur Ausatemöffnung (24) gas- und druckdicht ausgebildet, derart, daß bei geschlossenem Einatemventil (20) die in Richtung auf den Handapparatinnenraum strömende Ausatemluft zur Ausatemöffnung (24) hin ausweichen muß, wobei die Ausatemluft im wesentlichen nicht in den Handapparatinnenraum stromab der Ausatemöffnung (24) eindringt,
**gekennzeichnet durch folgende Merkmale:**
J. die Nebelkammer (3) besitzt zwischen ihren Enden eine gekrümmte Form,
K. die Nebelkammer (3) ist mit ihrem oberen Ende an das Mündungselement (4) angeschlossen,
L. die Einatemöffnung (19) ist in der Nebelkammer (3) zwischen deren Enden und oberhalb des Aufnahmegefäßes (9) angeordnet,
M. das Mündungselement (4) weist die Form eines T-Stücks auf und besitzt einen geradlinigen Längsabschnitt, der sich zwischen der Mündungsöffnung (22) und dem Anschluß des Mündungselements (4) an die Nebelkammer (3) erstreckt, sowie einen Querabschnitt, der die Ausatemöffnung (24) enthält,
N. eine Längsachse des Querabschnittes schließt auf einer von der Mündungsöffnung (22) abgewandten Seite einen Winkel mit einer Längsachse des Längsabschnittes ein, der etwa 90° beträgt oder kleiner als 90° ist.

2. Inhalator nach Anspruch 1,
**dadurch gekennzeichnet,**
**daß** in der Ausatemöffnung (24) ein Ausatemfilter (25) angeordnet ist, das für die vernebelte Flüssigkeit (15,16) undurchlässig ist.

3. Inhalator nach Anspruch 1 oder 2,
**dadurch gekennzeichnet,**
**daß** ein Einatemfilter (30) vorgesehen ist, das bezüglich einer Einatemströmung stromauf des Einatemventils (20) in der Einatemöffnung (19) angeordnet ist.

4. Inhalator nach einem der Ansprüche 1 bis 3,
**dadurch gekennzeichnet,**
**daß** die Ausatemöffnung (24) nahe der Mündungsöffnung (22) angeordnet ist.

5. Inhalator nach einem der Ansprüche 1 bis 4,
**dadurch gekennzeichnet,**
**daß** das Einatemventil (20) und das Ausatemventil (26) jeweils lösbar am Handapparat (1) befestigt sind, wobei die Ventile (20 und 26) verwechslungssicher ausgestaltet sind, so daß das Einatemventil (20) ausschließlich in der Einatemöffnung (19) und das Ausatemventil (26) ausschließlich in der Ausatemöffnung (24) in der jeweils vorgesehenen Durchströmungsrichtung montierbar sind.

6. Inhalator nach einem der Ansprüche 1 bis 5,
**dadurch gekennzeichnet,**
**daß** die Ausströmrichtung der Atemluft mit einem Winkel von etwa 60° gegenüber der Durchströmrichtung (29) geneigt verläuft.

7. Inhalator nach einem der Ansprüche 1 bis 6,
**dadurch gekennzeichnet,**
**daß** das durchströmte Innere des Handapparates (1) derart ausgebildet und die Einatemöffnung so positioniert und orientiert ist, daß die beim Ansaugen bzw. Einatmen erzeugte Einatemströmung bis zur Mündungsöffnung (22) laminar ist.

8. Inhalator nach einem der Ansprüche 1 bis 7,
**dadurch gekennzeichnet,**
**daß** der Durchströmungsquerschnitt für die Einatemströmung entlang ihres Verlaufes bis zur Mündungsöffnung (22) abnimmt.

9. Inhalator nach einem der Ansprüche 1 bis 8,
**dadurch gekennzeichnet,**
**daß** die der Atemluft und/oder der Flüssigkeit (14, 15, 16) ausgesetzten Verbindungen (5, 6 und 7, 8) zwischen den Einzelelementen (3, 4 und 2, 3) des Handapparates (1) druckdicht ausgebildet sind.

10. Inhalator nach Anspruch 9,
**dadurch gekennzeichnet,**
**daß** die Verbindungen (5, 6 und 7, 8) bis mindestens 500 mbar druckdicht ausgebildet sind.

11. Inhalator nach einem der Ansprüche 1 bis 10,
**dadurch gekennzeichnet,**
**daß** die lösbaren Verbindungen zwischen den Einzelelementen (2, 3 und 3, 4) des Handapparates (1) jeweils als Steckverbindung nach Art eines Bajonett-Verschlusses mit Stutzen (5, 7) und korrespondierender Aufnahme (6, 8) ausgebildet sind.

## Claims

1. An inhaler with a device for nebulizing liquids, having the following features:
A. a hand apparatus (1) of the inhaler has a handle element (2), a nebulizer chamber (3) and mouth element (4),
B. the handle element (2) contains in an upper section a receptacle container (9) which is open at the top to accommodate a liquid (14) which is to be nebulized,
C. the handle element (2) also contains a nebulizer (11) for nebulizing the liquid (14),
D. the nebulizer chamber (3) is connected at its lower end to the handle element (2) and communicates with the receptacle container (9),
E. the nebulizer chamber (3) contains an inhalation opening (19) through which fresh air enters from the environment (27) into the nebulizer chamber (3) on inhalation of air,
F. the mouth element (4) contains a mouth opening (22) and an exhalation opening (24) through which exhaled air enters the environment in exhalation,
G. an inhalation valve (20) which opens on inhalation and closes on exhalation is arranged in the inhalation opening (19),
H. an exhalation valve (26) which closes on inhalation and opens on exhalation is arranged in the exhalation opening (24),
I. the interior of the hand apparatus is designed to be airtight and pressure-proof at least up to the exhalation opening (24) such that when the inhalation valve (20) is closed the exhaled air flowing in the direction of the interior of the hand apparatus must escape toward the exhalation opening (24) whereby the exhaled air essentially does not penetrate into the interior of the hand apparatus downstream from the exhalation opening (24),
**characterized by** the following features:
J. the nebulizer chamber (3) has a curved shape between its ends,
K. the nebulizer chamber (3) is connected at its upper end to the mouth element (4),
L. the inhalation opening (19) is arranged in the nebulizer chamber (3) between its ends and above the receptacle container (9),
M. the mouth element (4) has the shape of a T-piece and has a linear longitudinal section which extends between the mouth opening (22) and the connection of the mouth element (4) to the nebulizer chamber (3), and it also has a transverse section which contains the exhalation opening (24),
N. a longitudinal axis of the transverse section forms an angle with a longitudinal axis of the longitudinal section amounting to approximately 90 degrees or less than 90 degrees on the side facing away from the mouth opening (22).

2. The inhaler according to Claim 1, **characterized in that** an exhalation filter (25) which is impermeable for the liquid (15, 16) nebulized is arranged in the exhalation opening (24).

3. The inhaler according to Claim 1 or 2, **characterized in that** an inhalation filter (30) is provided and is arranged upstream from the inhalation valve (20) with respect to an inhalation flow in the inhalation opening (19).

4. The inhaler according to one of Claims 1 through 3, **characterized in that** the exhalation opening (24) is arranged close to the mouth opening (22).

5. The inhaler according to one of Claims 1 through 4, **characterized in that** the inhalation valve (20) and the exhalation valve (26) are each detachably mounted on the hand apparatus (1), whereby the valves (20 and 26) are designed to be protected from improper mounting, i.e., the inhalation valve (20) can be mounted only in the inhalation opening (19) and the exhalation valve (26) can be mounted only in the exhalation opening (24), each in the respective flow-through direction provided for it.

6. The inhaler according to one of Claims 1 through 5, **characterized in that** the outflow direction of the respiratory air runs at an inclined angle of approximately 60 degrees to the through-flow direction (29).

7. The inhaler according to one of Claims 1 through 6, **characterized in that** the interior of the hand apparatus (1) through which the air flows is guided and the inhalation opening is positioned and oriented such that the inhalation flow created on intake or inhalation is laminar up to the mouth opening (22).

8. The inhaler according to one of Claims 1 through 7, **characterized in that** the flow-through cross section for the inhalation flow decreases along its course up to the mouth opening (22).

9. The inhaler according to one of Claims 1 through 8, **characterized in that** the connections (5, 6 and 7, 8) between the individual elements (3, 4 and 2, 3) of the hand apparatus (1) which are exposed to respiratory air and/or the liquid (14, 15, 16) are designed to be pressure-proof.

10. The inhaler according to Claim 9, **characterized in that** the connections (5, 6 and 7, 8) are designed to be pressure-proof up to at least 500 mbar.

11. The inhaler according to one of Claims 1 through 10, **characterized in that** the detachable connections between the individual elements (2, 3 and 3, 4) of the hand apparatus (1) are each designed as plug connections in the manner of a bayonet closure having nipples (5, 7) and a corresponding receptacle (6, 8).

## Revendications

1. Inhalateur comportant un dispositif de nébulisation de liquides, présentant les caractéristiques suivantes :
A. un appareil manuel (1) de l'inhalateur présente un corps de préhension (2), une cellule de nébulisation (3) et un élément à embouchure (4),
B. le corps de préhension (2) contient dans sa section supérieure un récipient récepteur ouvert vers le haut (9) destiné à recevoir un liquide à nébuliser (14),
C. le corps de préhension (2) contient de plus un nébuliseur (11) pour la nébulisation du liquide (14),
D. la cellule de nébulisation (3) se raccorde par son extrémité inférieure au corps de préhension (2) et communique avec le récipient récepteur (9),
E. la cellule de nébulisation (3) comporte une ouverture d'inspiration (19) à travers laquelle, lors de l'aspiration, de l'air frais en provenance de l'environnement (27) pénètre dans la cellule de nébulisation (3),
F. l'élément d'embouchure (4) contient une ouverture d'embouchure (22) ainsi qu'une ouverture d'expiration (24) à travers laquelle l'air expiré lors de l'expiration sort dans l'environnement,
G. dans l'ouverture d'inspiration (19) est disposée une soupape d'inspiration (20) qui s'ouvre à l'inspiration et se ferme à l'expiration,
H. dans l'ouverture d'expiration (24) est disposée une soupape d'expiration (26) qui se ferme à l'inspiration et s'ouvre à l'expiration,
I. l'espace intérieur de l'appareil manuel est conçu hermétique au gaz et à la pression du moins jusqu'à l'ouverture d'expiration (24) de manière à ce que, lorsque la soupape d'inspiration (20) est fermée, l'air expiré affluant en direction de l'espace intérieur de l'appareil manuel doive s'échapper vers l'ouverture d'expiration (24), l'air expiré ne pénétrant substantiellement pas dans l'espace intérieur de l'appareil manuel en aval de l'ouverture d'expiration (24),
**caractérisé par** les caractéristiques suivantes :
J. la cellule de nébulisation (3) possède entre ses extrémités une forme courbée,
K. la cellule de nébulisation (3) se raccorde par son extrémité supérieure à l'élément à embouchure (4),
L. l'ouverture d'inspiration (19) est disposée dans la cellule de nébulisation (3) entre les extrémités de celle-ci et au dessus du récipient récepteur (9),
M. l'élément à embouchure (4) présente la forme d'une pièce en T et possède une section longitudinale en ligne droite qui s'étend entre l'ouverture d'embouchure (22) et le raccordement de l'élément d'embouchure (4) à la cellule de nébulisation (3) ainsi qu'une section transversale qui comporte l'ouverture d'expiration (24),
N. un axe longitudinal de la section transversale forme sur un côté détourné de l'ouverture de l'embouchure (22) un angle avec un axe longitudinal de la section longitudinale, angle qui est d'environ 90° ou est inférieur à 90°.

2. Inhalateur selon la revendication 1, **caractérisé en ce que** dans l'ouverture d'expiration (24) est disposé un filtre d'expiration (25) qui est imperméable au liquide nébulisé (15, 16).

3. Inhalateur selon la revendication 1 ou 2, **caractérisé en ce que** il est prévu un filtre d'inspiration (30) qui est disposé, par rapport à un flux d'inspiration, en amont de la soupape d'inspiration (20) dans l'ouverture d'inspiration (19).

4. Inhalateur selon l'une quelconque des revendications 1 à 3, **caractérisé en ce que** l'ouverture d'expiration (24) est disposée près de l'ouverture de l'embouchure (22).

5. Inhalateur selon l'une quelconque des revendications 1 à 4, **caractérisé en ce que** la soupape d'inspiration (20) et la soupape d'expiration (26) sont respectivement fixées de manière amovible sur l'appareil manuel (1), les soupapes (20 et 26) étant conçues non interchangeables de manière à ce que la soupape d'inspiration (20) puisse être montée exclusivement dans l'ouverture d'inspiration (19) et la soupape d'expiration (26) exclusivement dans l'ouverture d'expiration (24) dans le sens de flux respectivement prévu.

6. Inhalateur selon l'une quelconque des revendications 1 à 5, **caractérisé en ce que** le sens de flux de l'air de respiration sortant est orienté en inclinaison dans un angle d'environ 60° par rapport au sens de flux (29).

7. Inhalateur selon l'une quelconque des revendications 1 à 6, **caractérisé en ce que** l'intérieur de l'appareil manuel (1) traversé par le flux est conformé et l'ouverture d'inspiration est positionnée et orientée de manière à ce que le flux d'inspiration généré lors de l'aspiration ou de l'inspiration soit laminaire jusqu'à l'ouverture de l'embouchure (22).

8. Inhalateur selon l'une quelconque des revendications 1 à 7, **caractérisé en ce que** la section transversale de flux décroît pour le flux d'inspiration le long de sa trajectoire vers l'ouverture de l'embouchure (22).

9. Inhalateur selon l'une quelconque des revendications 1 à 8, **caractérisé en ce que** les liaisons (5, 6 et 7, 8) exposées à l'air de respiration et/ou au liquide (14, 15, 16) entre les éléments individuels (3, 4 et 2, 3) de l'appareil manuel (1) sont conçues hermétiques à la pression.

10. Inhalateur selon la revendication 9, **caractérisé en ce que** les liaisons (5, 6 et 7, 8) sont conçues hermétiques à la pression jusqu'à au moins 500 mbar.

11. Inhalateur selon l'une quelconque des revendications 1 à 10, **caractérisé en ce que** les liaisons détachables entre les éléments individuels (2, 3 et 3, 4) de l'appareil manuel (1) sont conçues respectivement comme des cosses de raccordement à la manière d'une fermeture à baïonnette avec des tubulures (5, 7) et un support correspondant (6, 8).
